(19) Europäisches Patentamt
European Patent Office
Office européen des brevets

(11) Veröffentlichungsnummer: **0 260 486**
**B1**

(12) **EUROPÄISCHE PATENTSCHRIFT**

(45) Veröffentlichungstag der Patentschrift: **23.01.91**

(21) Anmeldenummer: **87112297.4**

(22) Anmeldetag: **25.08.87**

(51) Int. Cl.$^5$: **C 07 D 265/38,** C 12 P 17/14, A 61 K 31/535, C 12 N 1/20 // (C12P17/14, C12R1:465),(C12N1/20, C12R1:465)

# (54) Neue pharmakologisch wirksame Phenoxazinone, Verfahren zu ihrer Herstellung und ihre Verwendung.

(30) Priorität: **27.08.86 DE 3629062**

(43) Veröffentlichungstag der Anmeldung:
**23.03.88 Patentblatt 88/12**

(45) Bekanntmachung des Hinweises auf die Patenterteilung:
**23.01.91 Patentblatt 91/04**

(84) Benannte Vertragsstaaten:
**AT BE CH DE ES FR GB GR IT LI LU NL SE**

(56) Entgegenhaltungen:
**THE JOURNAL OF ANTIBIOTICS, Band 36, Nr. 6, Juni 1983, Seiten 688-694; N.N. GERBER et al.: "Structure and syntheses of texazone, 2-(N-methylamino)-3H-phenoxazin-3-one-8-carboxylic acid, an actionomycete metabolite"**

**THE JOURNAL OF ORGANIC CHEMISTRY, Band 32, Nr. 12, Dezember 1967, Seiten 4055-4057; N.N. GERBER: "Phenazines, phenoxazinones, and dioxopiperaines from Streptomyces thioluteus"**

(73) Patentinhaber: **HOECHST AKTIENGESELLSCHAFT**
**Postfach 80 03 20**
**D-6230 Frankfurt am Main 80 (DE)**

(72) Erfinder: **Zeeck, Axel, Prof., Dr.**
**Brüder-Grimm-Allee 22**
**D-3400 Göttingen (DE)**
Erfinder: **Breiding-Mack, Sabine, Dr.**
**Am Papenberg 13**
**D-3381 Gross Döhren (DE)**
Erfinder: **Grabley, Susanne, Dr.**
**Hölderlinstrasse 7**
**D-6240 Königstein/Taunus (DE)**
Erfinder: **Voelskow, Hartmut, Dr.**
**Akazienstrasse 22**
**D-6234 Hattersheim am Main (DE)**
Erfinder: **Seibert, Gerhard, Prof. Dr.**
**Gläserweg 21**
**D-6100 Darmstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 260 486 B1

**Beschreibung**

Aus einer Bodenprobe wurde ein Mikroorganismenstamm, der als Streptomyces spec. indentifiziert wurde, isoliert und bei der Deutschen Sammlung von Mikroorganismen (DSM) unter der Nummer DSM 3813 hinterlegt.

Dieser Stamm ist wie folgt charakterisiert:

| | |
|---|---|
| Sporenoberfläche: | Sm |
| Sporenmorphologie: | Rf |
| Chromogenität: | M+ |
| Luftmycelfarbe: | grau |

In einer eine Kohlenstoff- und Stickstoffquelle sowie die üblichen anorganischen Salze enthaltenden Nährlösung produziert der Stamm DSM 3813 neue Verbindungen mit Phenoxazinon-Grundgerüst. Diese Verbindungen sind also erhältlich durch Ferrmentation des Stammes DSM 3813 und Isolierung aus dem Mycel und insbesondere aus dem Fermentationsmedium.

Aus Veröffentlichungen ist zwar bekannt, daß Aktinomyceten ein Aminophenoxazinon synthetisieren [Gerber et al.: J. Antibiot., Seite 688 (1983); Gerber, N. N.: J. Org. Chem. *32*, 4055 (1967)] können. Die im folgenden aufgeführten, mikrobiologisch gewonnenen Phenoxazinone sind jedoch neu.

Die neuen Phenoxazinone wirken antifungisch, antiviral, als Anthelminthikum und als Hemmer der Lipoxygenase und können daher in Form pharmazeutischer Präparate zur Behandlung von Mensch und Tier bzw. im Diagnostikabereich Anwendung finden.

Die neuen Verbindungen können ferner als Zwischenprodukte zur Herstellung neuer Derivaten mit vergleichbarer Wirkung dienen.

Die Erfindung betrifft somit:

1. Die Verbindung der allgemeinen Formel I

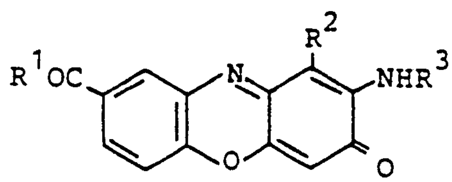

in der

$R^1$ Hydroxyl oder ($C_1$—$C_5$)-Alkoxy oder Amino

$R^2$ Wasserstoff oder die Gruppe der allgemeinen Formel

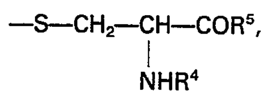

mit $R^4$ als Wassertoff oder ($C_1$—$C_5$)-Acyl und $R^5$ als Hydroxyl oder ($C_1$—$C_5$)-Alkoxy, und

$R^3$ Wasserstoff oder ($C_1$—$C_5$)-Acyl

bedeuten; ausgenommen die Verbindung 2-Amino-3H-phenoxazin-3-on-carbonsäureethylester.

2. Ein Verfahren zur Herstellung der Verbindungen der allgemeinen Formel I, das dadurch gekennzeichnet ist, daß

a) Streptomyces spec. DMS 3813 auf einem Nährboden kultiviert wird, bis sich die Verbindungen der allgemeinen Formel I, in denen

1) $R^1$ Hydroxyl, $R^2$

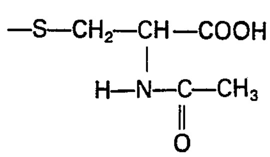

und $R^3$ Wasserstoff oder

2) $R^1$ Hydroxyl und $R^2$ Wasserstoff sowie $R^3$ Acetyl oder

3) $R^1$ Amino und $R^2$ und $R^3$ Wasserstoff bedeutet,

im Nährmedium anhäuft und gegebenenfalls

b) die genannten Verbindungen isoliert und derivatisiert werden.

3. Die Verwendung der genannten Verbindungen der allgemeinen Formel I zur Herstellung von Heilmitteln oder von Diagnostika.

Im folgenden wird die Erfindung im einzelnen erläutert bzw. in den Patentansprüchen definiert.

Die Verbindungen der allgemeinen Formel I, in denen

a) $R^1$ Hydroxyl, $R^2$

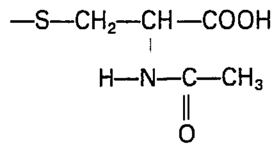

und $R^3$ Wasserstoff oder

b) $R^1$ Hydroxy und $R^2$ Wasserstoff sowie $R^3$ Acetyl oder

c) $R^1$ Amino und $R^2$ und $R^3$ Wasserstoff bedeutet,

können fermentativ mit Hilfe von Streptomyces spec. DSM 3813 hergestellt werden.

Anstelle des Stammes DMS 3813 können natürlich auch seine Mutanten und Varianten eingesetzt werden, soweit sie die genannte Verbindung produzieren. Solche Mutanten können in an sich bekannter Weise durch physikalische Mittel, beispielsweise Bestrahlung, wie mit Ultraviolett- oder Röntgenstrahlen, oder chemische Mutagene, wie beispielsweise Ethansulfonsäuremethylester (Ethylmethylsulfonat, EMS) oder 2-Hydroxy-4-methoxy-benzophenon (MOB), erzeugt werden.

Als bevorzugte Kohlenstoffquellen für die aerobe Fermentation eignen sich assimilierbare Kohlenhydrate und Zuckeralkohole, wie Glukose, Lactose oder D-Mannit, sowie kohlenhydrathaltige Naturprodukte, wie Malzextrakte. Als stickstoffhaltige Nährstoffe kommen in Betracht: Aminosäuren, Proteine und deren Abbauprodukte, wie Peptone oder Tryptone, ferner Fleischextrakte, gemahlene Samen, beispielsweise von Mais, Weizen, Bohnen, Soja oder der Baumwollpflanze, Destillationsrückstände der Alkoholherstellung Fleischmehle oder Hefeextrakte, aber auch Ammoniumsalze und Nitrate. An anderen anorganischen Salzen kann die Nährlösung beispielweise Chloride, Carbonate, Sulfate oder Phosphate der Alkali- oder Erdalkalimetalle, Eisen, Zink und Mangan enthalten.

Die Bildung der fermentativ herstellbaren Verbindungen Ia, b, c verläuft besonders gut in einer Nährlösung, die etwa 2% Sojamehl und 2% Mannit, jeweils bezogen auf das Gewicht der gesamten Nährlösung, enthält.

Die Fermentation erfolgt aerob, also beispielsweise submers unter Schüttlen oder Rühren in Schüttelkolben oder Fermentern, gegebenenfalls unter Einführung von Luft oder Sauerstoff. Die Fermentation kann in einem Temperaturbereich von etwa 18 bis 35°C, vorzugsweise bei etwa bis 30°C, insbesondere bei 28 bis 30°C erfolgen. Es wird in einem pH-Bereich zwischen 5 und 8,5, vorzugsweise zwischen 5,5 und 8,0 fermentiert. Unter diesen Bedingungen zeigt die Kulturbrühe im allgemeinen nach 2 bis 6 Tagen eine nennenswerte Akkumulation der Verbindungen Ia, b, c.

Vorteilhaft kultiviert man in mehreren Stufen, d.h. man stellt zunächst eine oder mehrere Vorkulturen in einem flüssigen Nährmedium her, die dann in das eigentliche Produktionsmedium, die Hauptkultur, beispielsweise im Volumenverhältnis 1:10, überimpft werden. Die Vorkultur erhält man z.B., indem man ein versportes Mycel in eine Nährlösung überimpft und etwa 48 bis 72 Stunden wachsen läßt. Das versporte Mycel kann erhalten werden, indem man den Stamm etwa 7 Tage auf einem festen oder flüssigen Nährboden, beispielsweise Hefe-Malz-Agar kultiviert.

Der Fermentationsverlauf kann anhand des pH-Wertes der Kultur, des Mycelvolumens oder durch Dünnschicht-Chromatographie überwacht werden.

Die Isolierung der fermentativ herstellbaren Verbindungen Ia, b, c aus der Kultur erfolgt nach bekannten Methoden unter Berücksichtigung der chemischen, physikalischen und biologischen Eigenschaften der Produkte.

Es ist sinnvoll zuvor eine Detektion über Dünnschichtchromatographie auf Kieselgel mit einem polaren Lösungsmittel bzw. Lösungsmittelgemisch, beispielsweise n-Butanol/Essigsäure/Wasser als Laufmittel, durchzuführen.

Die Verbindungen können aus der unfiltrierten Kulturbrühe mit einem mit Wasser nicht oder nur wenig mischbaren organischen Lösemittel, wie n-Butanol, Aceton, Methanol etc. extrahiert werden, zweckmäßig bei pH 5 bis 6. Alternativ läßt sich die lyophilisierte Kultur mit Methanol oder anderen niederen Alkanolen extrahieren, um die Verbindungen roh zu isolieren.

Man erhält farbige Extrakte, aus denen nach Abtrennen der lipophilen Bestandteile durch Extraktion mit entsprechenden Lösungsmitteln, beispielsweise mit einem Gemisch aus Chloroform/Petrolether, und Auftrennen des Rohprodukts durch Chromatographie 3 Fraktionen erhalten werden.

Zur Isolierung der einzelnen Fraktionen aus dem entfetteten Rohextrakt wird dieser zweckmäßig über Hydroxyalkoxypropyldextran (®Sephadex LH-Marken) gereinigt, wobei sich als Fließmittel Gemische aus niederen Alkanolen und Wasser, z.B. Methanol und Wasser im Volumenverhältnis 9:1, oder reines Methanol bewährt haben. Die in Form verschiedenfarbiger Zonen adsorbierten Komponenten werden nacheinander in Form verschiedenfarbiger Eluate isoliert, in denen jeweils eine der Komponenten stark angereichert ist.

Zur Isolierung der reinen Verbindungen können die üblichen Verfahrensschritte Verwendung finden, wie Chromatographie oder erneute Gelfiltration in geeigneten organischen Lösemitteln. Besonders bewährt hat sich eine Chromatographie an Sephadex LH-Marken, wobei als Laufmittel besonders reines Methanol oder Methanol/$H_2O$ im Verhältnis 9:1 bis 90:1 Verwendung finden.

Die reinen Verbindungen Ia, b, c sind amorphe bis kristalline Feststoffe ohne pH-Indikatoreigenschaften. Die Verbindungen sind in stark polaren Lösemitteln, wie Methanol, DMSO und Wasser gut löslich, mäßig löslich in Ethanol, Chloroform und Dichlormethan. Sie sind unlösich in Alkanen und Aceton.

Die Überführung in die Derivate der Verbindungen Ia, b, c erfolgt in an sich bekannter Weise. Ia und Ib können beispielsweise durch Methanolyse in Gegenwart starker Säuren in die Ester übergeführt werden. Geeignet sind z.B. Gemische aus Methanol und wasserfreier Salzsäure, in denen die Methanolyse in 1 Stunde bis 24 Stunden bei Temperaturen von 20 bis 60°C, insbesondere bei Zimmertemperatur, vollständig abläuft.

Die Abspaltung des Acetylrestes aus Ia erfolgt in an sich bekannter Weise, vorzugsweise mittels Aminoacylasen in wäßrigem Medium.

Von besonderem Interesse ist die pharmakologische Wirkung der Verbindungen gegen Viren, beispielsweise gegen Vaccina P71 in Helza-Zellen und Influenza A in Vero-Zellen, sowie gegen Protozoen wie Trichomonas vaginalis und Pilze wie Candida albicans. Außerdem können die Produkte vorteilhaft als Anthelminthikum eingesetzt werden. Sie sind insbesondere wirksam gegen eine große Anzahl von Helminthen, z.B. Hemonchus, Trichostrongylus, Ostertagia, Cooperia, Chabertia, Strongyloides, Oesophagostomum, Hysostrongylus, Ancylostoma, Ascaris und Heterakis. Besonders ausgeprägt ist die Wirksamkeit gegenüber Magen-Darm-Strongyliden und Lungenwürmern, von denen vor allem Haus- und Nutztiere befallen werden. Deshalb werden die Verbindungen gemäß der Erfindung insbesondere in Tierarzneimitteln verwendet.

Die Substanzen sind in fasten Zustand und in Lösungen im pH-Bereich von 2 bis 8, insbesondere von 5 bis 8, stabil. Die Verbindungen lassen sich damit in übliche galenische Zubereitungen einarbeiten.

In den folgenden Beispielen wird die Erfindung näher erläutert. Prozentangaben beziehen sich auf das Gewicht, wenn nichts anderes angegeben ist. Mischungsverhältnisse bei Flüssigkeiten beziehen sich auf das Volumen, sofern keine anderen Angaben gemacht sind.

## Beispiel 1

a) Herstellung einer Sporensuspension des Produzentenstammes Streptomyces spec. DSM 3813

Mehrere Schrägröhrchen mit Haferflockenagar werden mit DSM 3813 beimpft und ca. 6 Tage bei 30°C inkubiert. Der Haferflockenagar wird wie folgt hergestellt: 20 g Haferflocken werden in 1 l Wasser 20 min. gekocht und anschließend filtriert. Dem Filtrat werden 18 g Agar und 2,5 ml einer Spurenelementlösung (3 g $CaCl_2 \cdot 2H_2O$, 1 g Fe-III-Citrat, 0,2 g $MnSo_4$, 0,1 g $ZnCl_2$, 0,025 g $CuSo_4 \cdot 5H_2O$, 0,02 g $Na_2B_4O_7 \cdot 10H_2O$, 0,004 g $CaCl_2$ und 0,1 g $Na_2M_6O_4 \cdot H_2O$ in 1 l dest. Wasser gelöst) zugegeben. Vor der Sterilisation wird ein pH-Wert von 7,8 eingestellt.

Die Sporen eines jeden Röhrchens werden mit 3 ml Flüssigkeit [0,9% NaCl, 0,1% Polyoxyethylensorbitanmonooleat (®Tween 80) in destilliertem Wasser] abgeschwemmt. Die Suspensionen werden vereinigt und bis zur Beimpfung der Hauptkultur bei 4°C aufbewahrt.

b) Herstellung einer Vorkultur des Produzentenstammes, DSM 3813, im Erlenmeyerkolben

5 Erlenmeyerkolben (300 ml Fassungsvermögen) mit je 100 ml Nährlösung (40 g Glukose, 30 g Sojamehl, 2,5 g NaCl, 2,5 g $CaCO_3$ auf 1 l destilliertes Wasser, pH 7,5 vor der Sterilisation eingestellt) werden mit je 1,5 ml der möglichst frischen Sporensuspension angeimpft und auf einer Schüttelmaschine 5 (180 UPM) bei 30°C 72 Stunden inkubiert.

c) Herstellung der Verbindungen Ia, b, c mit einer Kultur des Produzentenstammes DSM 3813

Ein 300 ml Erlenmeyerkolben mit 100 ml Nährlösung (20 g Sojamehl, 20 g Mannit auf 1 l destilliertes Wasser, pH 7,5 vor der Sterilisation eingestellt) wird mit 3 ml der Vorkultur beimpft und bei 30°C auf der Schüttelmaschine (180 UPM) inkubiert. Das Proudktionsmaximum wird nach ca. 96 bis 120 Stunden erreicht. Die Gesamtausbeuten der Verbindungen Ia, b, c liegen bei ca. 200 mg/l.

## Beispiel 2

Kultivierung des Produzentenstammes, DSM 3813, im Fermenter

Ein Fermenter mit 13 l Fassungsvermögen wird unter folgenden Bedingungen betrieben: Bei 30°C Inkubationstemperatur und 600 UPM des Rührers werden 10 l Luft pro Minute in die Kulturflüssigkeit (Medium wie in Beispiel 1c) eingeleitet. Der Fermenter wird mit 500 ml der Vorkultur (siehe 1b) beimpft. Das Produktionsoptimum wird nach ca. 96 bis 120 Stunden erreicht.

Die Gesamtausbeuten der Verbindungen Ia, b, c liegen bei ca. 400 mg/l.

## Beispiel 3

Isolierung der Verbindungen Ia, b, c

60 g Lyophilisat aus 4 l Kulturfiltrat des Stammes DSM 3813 werden 3 mal mit 1 l Methanol bei pH 5 bis 6 extrahiert und im Vakuum eingedampft. Den Rückstand nimmt man in Methanol/Waser (9:1, V:V) auf und

chromatographiert in mehreren Portionen an ®Sephadex LH 200 mit Methanol/Wasser 9:1. Die Verbindungen Ib und Ia werden zunächst zusammen als langgezogene gelb- bis rotbraune Zone eluiert, anschließend folgt als braunrote Zone die Verbindung Ic. Die aus mehreren Sephadex-Trennung erhaltenen Fraktionen Ia und Ib werden erneut an Sephadex LH 20 mit Methanol chromatographiert, wobei Ib vor Ia eluiert wird. Ein großer Teil Ib bleibt beim Lösen des Gemisches ungelöst zurück und wird abfiltriert. Insgesamt erhält man 70 mg rotes, amorphes Ia und 14 mg gelbes, zum Teil kristallines Ib. Die Ic enthaltenden Fraktionen werden nochmals an 2 präparativen Kieselgel-Dickschichtplatten (20 × 20 cm) im System Ethylacetat/Methanol/Wasser 6:2:1 aufgetrennt. Die orangerote Hauptzone mit Chloroform/Methanol 1:1 wird eluiert, so daß man nach einer weiteren Reinigung an Sephadex LH 20 mit p.A. Methanol 7,5 mg Ic erhält.

Beispiel 4

Herstellung des Methylesters aus der Verbindung Ib

10 mg Ib werden mit 20 ml frisch hergestellter 5 molarer methanolischer Sälzsäure versetzt und 24 Stunden bei Raumtemperatur gerührt. Man dampft die Lösung im Vakuum ein, chromatographiert den Rückstand an Sephadex LH 20 mit Methanol/Chloroform 9:1 und erhält 5,5 mg Ib-Methylester.

Beispiel 5

Herstellung des Methylesters aus der Verbindung Ia

50 mg Ia werden mit 15 ml frisch hergestellter 5 molarer methanolischer Salzsäure versetzt und 24 Stunden bei Raumtemperatur gerührt. Nach dem Eindampfen im Vakuum chromatographiert man an Sephadex LH 20 (Säule 2,5 × 40 cm) mit Methanol und erhält 29 mg Ia-Dimethylester.

Spektroskopische Daten der Verbindungen Ia, b, c:

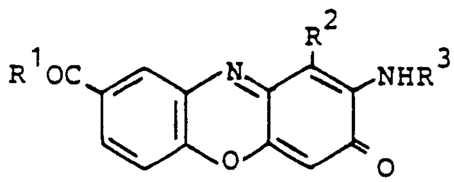

I a, b, c

| Verbindung | Gruppen $R^1$ | $R^2$ | $R^3$ |
|---|---|---|---|
| Ia | OH | $-S-CH_2-CH-COOH$<br>\|<br>$HN-C-CH_3$<br>‖<br>O | H |
| Ib | OH | H | $-OC-CH_3$ |
| Ic | $-NH_2$ | H | H |

| | Ia | Ib | Ic |
|---|---|---|---|
| UV [nm] | 428 | 402 | 429 |
| | 254 | 250 | 256 |
| IR [$cm^{-1}$] (KBr) | 3390 | 3425 | 3420 |
| | 3305 | 3295s | 3325 |
| | 1725w | | |
| | 1692m | 1687m | |
| | 1640m | 1618s | |
| | 1580 | 1588 | 1590s |
| | | | 1555 |
| | | 1510s | 1562 |
| | | | 1393s |

Ia: $^1$H-NMR: 200 MHz, $d_6$-DMSO = 1,80 (s, 3H); 3,17 (d, 2H); 4,35 (dd, 1H); 6,48 (s, 1H); 7,22 (breit, $NH_2$); 7,63 (d, 1H); 8,04 (dd, 1H); 8,25 (d, NH); 8,30 (d, 1H) ppm.

$^{13}$C-NMR: 50 MHz, $CD_3OD$ = 22,9 ($CH_3$); 37,5 ($CH_2$); 56,6 (CH); 102,1 (C-1); 105,0 (C-4); 116,2 C-6); 130,7; 131,9 (C-7, C-9); 134 (C-9a); 136,3 (C-8); 144,9 (C-5a); 147,8 (C-10a); 151,2 (C-4a); 152,2 (C-2); 173,0; 173,6 (C-4', C-5'); 177,1 (C-3) 180,5 (C-1') ppm.

Ib: $^1$H-NMR: 200 MHz, 60°C, $d_6$-DMSO = 2,25 (s, 3H); 6,52 (s, 1H); 7,57 (d, 1H); 8,16 (dd, 1H); 8,31 (s, 1H); 8,32 (d, 1H); 9,65 (1H, NH) ppm.

$^{13}$C-NMR: 50 MHz, 60°C, $d_6$-DMSO = 24,1 ($CH_3$); 103,9; 113,0; 115,4; 129,8; 132,1; 132,7; 137,5; 144,5; 148,4; 148,9; 166,0; 170,2; 179,2 ppm.

Beispiel 6

Anthelminthische Wirkung der Verbindung Ia

Die anthelminthische Wirkung der Ammoniumsalze der Verbindung Ia wird an Lämmern mit 30—40 kg Körpergewicht untersucht. Dazu werden die Lämmer artifiziell mit Infektionsstadien von Labmagen- (Haemunchus contortus) und Dünndarm- (Trichostrongylus colubriformis) Nematoden infiziert. Nach Abschluß der Entwicklungszeit (Präpatenzperiode) der Nematoden erfolgt die Applikation von Ia in einer Dosierung von 2,5 mg/kg (subcutan).

Durch koproskopische Untersuchungen vor und bis zu 14 Tagen nach der Applikation und anschließender Sektion mit helminthologischer Aufarbeitung wird die prozentuale Reduzierung der Schaf-Nematoden ermittelt. Es kann eine 80 bis 90 %ige Reduzierung festgestellt werden.

**Patentansprüche für die Vertragsstaaten: BE CH DE FR GB IT LI LU NL SE**

1. Verbindung der allgemeinen Formel I

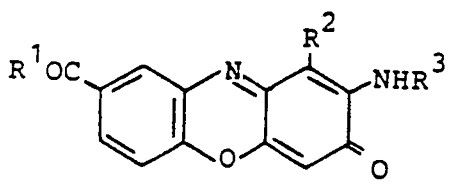

in der

$R^1$ Hydroxyl oder ($C_1$—$C_5$)-Alkoxy oder Amino

$R^2$ Wasserstoff oder die Gruppe der allgemeinen Formel

$—S—CH_2—CH—COR^5$,

$NHR^4$

mit $R^4$ als Wasserstoff oder ($C_1$—$C_5$)-Acyl und $R^5$ als Hydroxyl oder ($C_1$—$C_5$)-Alkoxy, und

$R^3$ Wasserstoff oder ($C_1$—$C_5$)-Acyl bedeuten, ausgenommen 2-Amino-3H-phenoxazin-3-on carbonsäureethylester.

2. Verfahren zur Herstellung der Verbindung der allgemeinen Formel I

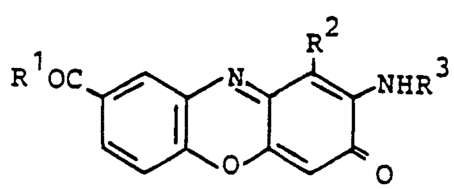

in der

$R^1$ Hydroxyl oder ($C_1$—$C_5$)-Alkoxy oder Amino

$R^2$ Wasserstoff oder die Gruppe der allgemeinen Formel

$—S—CH_2—CH—COR^5$,

$NHR^4$

mit $R^4$ als Wasserstoff oder ($C_1$—$C_5$)-Acyl mit $R^5$ als Hydroxyl oder ($C_1$—$C_5$)-Alkoxy, und

$R^3$ Wasserstoff oder ($C_1$—$C_5$)-Acyl bedeuten, dadurch gekennzeichnet, daß

a) Streptomyces spec. DMS 3813 auf einem Nährboden kultiviert wird, bis sich die Verbindung der allgemeinen Formel I, in der

1) $R^1$ Hydroxyl, $R^2$

$-S-CH_2-CH-COOH$
|
$H-N-C-CH_3$
||
O

und $R^3$ Wasserstoff oder

2) $R^1$ Hydroxyl und $R^2$ Wasserstoff sowie $R^3$ Acetyl oder

3) $R^1$ Amino und $R^2$ und $R^3$ Wasserstoff bedeutet, im Nährmedium anhäuft und gegebenenfalls

b) die genannten Verbindungen isoliert und derivatisiert werden.

3. Verfahren nach Anspruch 2, dadurch gekennzeichnet, daß Streptomyces spec., DSM 3813, in einer Nährlösung mit Sojamehl und Mannit kultiviert wird.

4. Verfharen nach Anspruch 2 oder 3, dadurch gekennzeichent, daß Streptomyces spec., DSM 3813, bei 18 bis 35°C kultiviert wird.

5. Verfahren nach einem oder mehreren der Ansprüche 2 bis 4, dadurch gekennzeichnet, daß Streptomyces spec. DSM 3813 bei einem pH-Wert von 5 bis 8,5 kultiviert wird.

6. Verfahren nach einem oder mehreren der Ansprüche 2 bis 5, dadurch gekennzeichnet, daß die fermentativ hergestellten Verbindungen chemisch derivatisiert werden.

7. Verwendung der Verbindung der allgemeinen Formel I

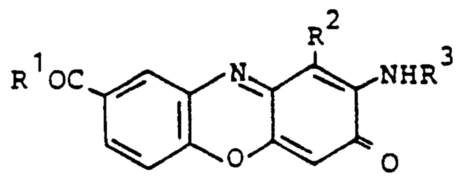

in der

$R^1$ Hydroxyl oder $(C_1-C_5)$-Alkoxy oder Amino

$R^2$ Wasserstoff oder die Gruppe der allgemeinen Formel

$-S-CH_2-CH-COR^5,$
|
$NHR^4$

mit $R^4$ als Wasserstoff oder $(C_1-C_5)$-Acyl und $R^5$ als Hydroxyl oder $(C_1-C_5)$-Alkoxy, und

$R^3$ Wasserstoff oder $(C_1-C_5)$-Acyl bedeuten, zur Herstellung von Heilmitteln oder Diagnostika.

8. Verwendung der Verbindung nach Anspruch 7 zur Herstellung 7 von Heilmitteln zur Behandlung von Pilz-, Protozoen- und viralen Infektionen sowie zur Herstellung von Anthelminthika und als Lipoxygenase Hemmstoff.

9. Verbindung nach Anspruch 17 zur Anwendung als Agens gegen Pilze, Viren, Protozoen und Helminthen.

10. Streptomyces spec. DSM 3813, seine Varianten und Mutanten, sofern sie die Verbindung der allgemeinen Formel I

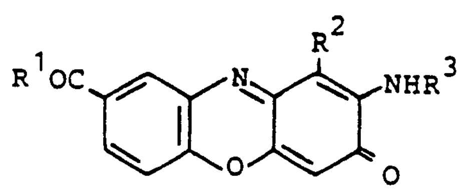

in der

$R^1$ Hydroxyl oder $(C_1-C_5)$-Alkoxy oder Amino

$R^2$ Wasserstoff oder die Gruppe der allgemeinen Formel

$-S-CH_2-CH-COR^5,$
|
$NHR^4$

mit $R^4$ als Wasserstoff oder $(C_1-C_5)$-Acyl mit $R^5$ als Hydroxyl oder $(C_1-C_5)$-Alkoxy, und

$R^3$ Wasserstoff oder $(C_1-C_5)$-Acyl bedeuten, produzieren.

**Patentansprüche für die Vertragsstaaten: AT ES GR**

1. Verfahren zur Herstellung der Verbindung der allgemeinen Formel I

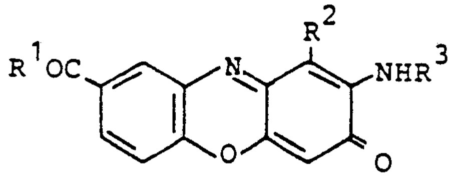

in der

$R^1$ Hydroxyl oder ($C_1$—$C_5$)-Alkoxy oder Amino

$R^2$ Wasserstoff oder die Gruppe der allgemeinen Formel

$$-S-CH_2-\underset{\displaystyle NHR^4}{\underset{|}{CH}}-COR^5,$$

mit $R^4$ als Wasserstoff oder ($C_1$—$C_5$)-Acyl und $R^5$ als Hydroxyl oder ($C_1$—$C_5$)-Alkoxy, und

$R^3$ Wasserstoff oder ($C_1$—$C_5$)-Acyl bedeuten, dadurch gekennzeichnet, daß

a) Streptomyces spec. DMS 3813 auf einem Nährboden kultiviert wird, bis sich die Verbindungen der allgemeinen Formel I, in denen

1) $R^1$ Hydroxyl, $R^2$

$$-S-CH_2-\underset{\displaystyle H-N-\underset{\underset{\displaystyle O}{\|}}{C}-CH_3}{\underset{|}{CH}}-COOH$$

und $R^3$ Wasserstoff oder

2) $R^1$ Hydroxyl und $R^2$ Wasserstoff sowie $R^3$ Acetyl oder

3) $R^1$ Amino und $R^2$ und $R^3$ Wasserstoff bedeutet, im Nährmedium anhäuft und gegebenenfalls

b) die genannten Verbindungen isoliert und derivatisiert werden.

2. Verfahren nach Anspruch 1, dadurch gekennzeichnet, daß Streptomyces spec., DSM 3813, in einer Nährlösung mit Sojamehl und Mannit kultiviert wird.

3. Verfahren nach Anspruch 1 oder 2, dadurch gekennzeichent, daß Streptomyces spec., DSM 3813, bei 18 bis 35°C kultiviert wird.

4. Verfahren nach einem oder mehreren der Ansprüche 1 bis 3, dadurch gekennzeichnet, daß Streptomyces spec. DSM 3813 bei einem pH-Wert von 5 bis 8,5 kultiviert wird.

5. Verfahren nach einem oder mehreren der Ansprüche 1 bis 4, dadurch gekennzeichnet, daß die fermentativ hergestellten Verbindungen chemisch derivatisiert werden.

6. Verwendung der Verbindung, erhältlich nach Anspruch 1, zur Herstellung von Heilmitteln oder Diagnostika.

7. Verwendung der Verbindung, erhältlich nach Anspruch 1, zur Herstellung von Heilmitteln zur Behandlung von Pilz-, Protozoen- und viralen Infektionen sowie zur Herstellung von Anthelminthika und als Lipoxygenase Hemmstoff.

8. Verbindung, erhältlich nach Anspruch 1, zur Anwendung als Agens gegen Pilze, Protozoen, Viren sowie Helminthen.

**Revendications pour les Etats contractants: BE CH DE FR GB IT LI LU NL SE**

1. Composé de formule générale I

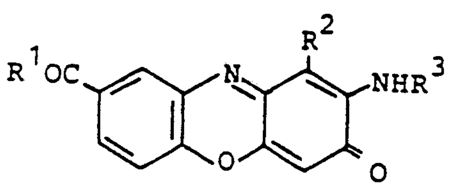

dans laquelle

$R^1$ est hydroxy ou alcoxy en $C_1$—$C_5$ ou amino,

$R^2$ est hydrogène ou le groupe de formule générale

$$—S—CH_2—CH(—NHR^4)—COR^5$$

avec $R^4$ = hydrogène ou acyle en $C_1—C_5$, $R^5$ = hydroxy ou alcoxy en $C_1—C_5$, et

$R^3$ est hydrogène ou acyle en $C_1—C_5$,

à l'exclusion de l'ester éthylique de l'acide 2-amino-3H-phénoxazine-3-onecarboxylique.

2. Procédé de préparation du composé de formule générale I

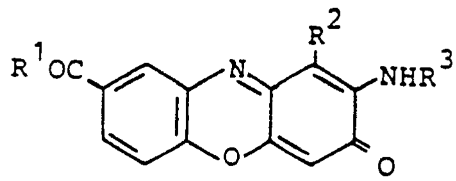

dans laquelle

$R^1$ est hydroxy ou alcoxy en $C_1—C_5$ ou amino,

$R^2$ est hydrogène ou le groupe de formule générale

$$—S—CH_2—CH(—NHR^4)—COR^5$$

avec $R^4$ = hydrogène ou acyle en $C_1—C_5$, $R^5$ = hydroxy ou alcoxy en $C_1—C_5$, et

$R^3$ est hydrogène ou acyle en $C_1—C_5$,

caractérisé en ce que

(a) on cultive Streptomyces spec, DMS 3813, sur un milieu de culture, jusqu'à accumulation dans le milieu de culture du composé de formule générale I, dans laquelle

1) $R^1$ est hydroxyle, $R^2$ représente le groupe

$$—S—CH_2—CH(—COOH)—NHCOCH_3$$

et $R^3$ est hydrogène, ou

2) $R^1$ est hydroxy, $R^2$ est hydrogène et $R^3$ est acétyle, ou

3) $R^1$ est amino et $R^2$ et $R^3$ sont hydrogène, et éventuellement

b) on isole les composés mentionnés et on en fait des dérivés.

3. Procédé selon la revendication 2, caractérisé en ce que l'on cultive Streptomyces spec., DSM 3813, sur un milieu de culture avec farine de soja et mannitol.

4. Procédé selon l'une des revendications 2 ou 3, caractérisé en ce que l'on cultive Streptomyces spec., DSM 3813, à une température allant de 18 à 35°C.

5. Procédé selon une ou plusieurs des revendications 2 à 4, caractérisé en ce que l'on cultive Streptomyces spec., DSM 3813, à un pH de 5 à 8,5.

6. Procédé selon une ou plusieurs des revendications 2 à 5, caractérisé en ce que l'on fait des dérivés chimiques des composés produits par fermentation.

7. Emploi du composé de formule générale I

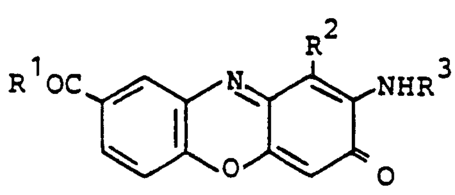

dans laquelle

$R^1$ est hydroxy ou alcoxy en $C_1—C_5$ ou amino,

$R^2$ est hydrogène ou le groupe de formule générale

$$—S—CH_2—CH(—NHR^4)—COR^5$$

avec $R^4$ = hydrogène ou acyle en $C_1—C_5$, $R^5$ = hydroxy ou alcoxy en $C_1—C_5$, et

$R^3$ est hydrogène ou acyle en $C_1—C_5$,

pour la fabrication de médicaments ou de produits pour diagnostic.

8. Emploi du composé selon la revendication 7 pour la fabrication de médicaments pour le traitement d'infections fongiques, par protozoaires et virales ainsi que pour la fabrication d'anthelminthiques et comme inhibiteur de lipoxygénase.

9. Composé selon la revendication 7 en tant qu'agent contre les champignons, les virus, les protozoaires et les helminthes.

10. Streptomyces spec. DSM 3813, ses variantes et mutants, dans la mesure où ils produisent le composé de formule générale

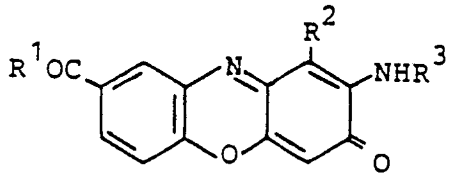

dans laquelle

$R^1$ est hydroxy ou alcoxy en $C_1$—$C_5$ ou amino,

$R^2$ est hydrogène ou le groupe de formule générale

$$—S—CH_2—CH(—NHR^4)—COR^5$$

avec $R^4$ = hydrogène ou acyle en $C_1$—$C_5$, $R^5$ = hydroxy ou alcoxy en $C_1$—$C_5$, et

$R^3$ est hydrogène ou acyle en $C_1$—$C_5$.

**Revendications pour les Etats contractants: AT ES GR**

1. Procédé de préparation du composé de formule générale I

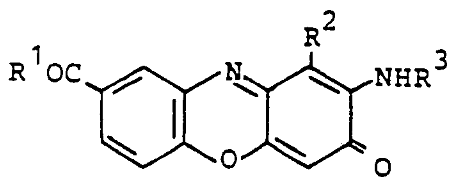

dans laquelle

$R^1$ est hydroxy ou alcoxy en $C_1$—$C_5$ ou amino,

$R^2$ est hydrogène ou le groupe de formule générale

$$—S—CH_2—CH(—NHR^4)—COR^5$$

avec $R^4$ = hydrogène ou acyle en $C_1$—$C_5$, $R^5$ = hydroxy ou alcoxy en $C_1$—$C_5$, et

$R^3$ est hydrogène ou acyle en $C_1$—$C_5$,

caractérisé en ce que

(a) on cultive Streptomyces spec, DMS 3813, sur un milieu de culture, jusqu'à accumulation dans le milieu de culture du composé de formule générale I, dans laquelle

1) $R^1$ est hydroxyle, $R^2$ représente le groupe

$$—S—CH_2—CH(—COOH)—NHCOCH_3$$

et $R^3$ est hydrogène, ou

2) $R^1$ est hydroxy, $R^2$ est hydrogène et $R^3$ est acétyle, ou

3) $R^1$ est amino et $R^2$ et $R^3$ sont hydrogène, et éventuellement

b) on isole les composés mentionnées et on en fait des dérivés.

2. Procédé selon la revendication 1, caractérisé en ce que l'on cultive Streptomyces spec., DSM 3813, sur un milieu de culture avec farine de soja et mannitol.

3. Procédé selon l'une des revendications 1 ou 2, caractérisé en ce que l'on cultive Streptomyces spec., DSM 3813, à une température allant de 18 à 35°C.

4. Procédé selon une ou plusieurs des revendications 1 à 3, caractérisé en ce que l'on cultive Streptomyces spec. DSM 3813, à un pH de 5 à 8,5.

5. Procédé selon une ou plusieurs des revendications 1 à 4, caractérisé en ce que l'on fait des dérivés chimiques des composés produits par fermentation.

6. Emploi du composé obtenu selon la revendication 1 pour la fabrication de médicaments ou de produits de diagnostic.

7. Emploi du composé obtenu selon la revendication 1 pour la fabrication de médicaments pour le traitement d'infections fongiques, par protozoaires et virales ainsi que pour la fabrication d'anthelminthiques et comme inhibiteur de lipoxygénase.

8. Composé obtenu selon la revendication 1, utilisé en tant qu'agent contre les champignons, les virus, les protozoaires et les helminthes.

**Claims for the Contracting States: BE CH DE FR GB IT LI LU NL SE**

1. A compound of the formula I

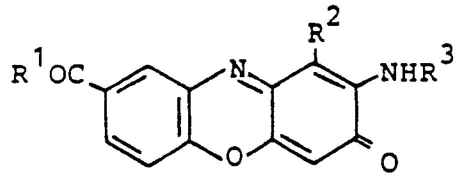

in which
$R^1$ is hydroxyl or $(C_1—C_5)$-alkoxy or amino,
$R^2$ is hydrogen or the group of the formula

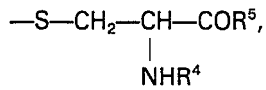

with $R^4$ as hydrogen or $(C_1—C_5)$-acyl and $R^5$ as hydroxyl or $(C_1—C_5)$-alkoxy, and
$R^3$ is hydrogen or $(C_1—C_5)$-acyl, with the exception of ethyl 2-amino-3H-phenoxazin-3-onecarboxylate.

2. A process for the preparation of a compound of the formula I

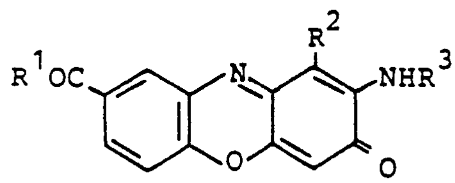

in which
$R^1$ is hydroxyl or $(C_1—C_5)$-alkoxy or amino,
$R^2$ is hydrogen or the group of the formula

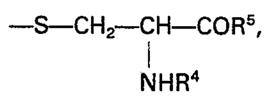

with $R^4$ as hydrogen or $(C_1—C_5)$-acyl and $R^5$ as hydroxyl or $(C_1—C_5)$-alkoxy, and
$R^3$ is hydrogen or $(C_1—C_5)$-acyl,
which comprises
a) culturing Streptomyces spec. DMS 3813 on a nutrient medium until a compound of the formula I in which

1) $R^1$ is hydroxyl, $R^2$ is

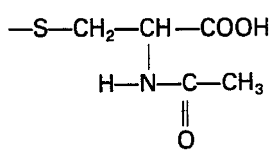

and $R^3$ is hydrogen, or
2) $R^1$ is hydroxyl, $R^2$ is hydrogen and $R^3$ is acetyl, or
3) $R^1$ is amino and $R^2$ and $R^3$ are hydrogen, has accumulated in the nutrient medium and if appropriate
b) isolating the compound mentioned and forming derivatives.

3. The process as claimed in claim 2, wherein Streptomyces spec. DS 3813 is cultured in a nutrient solution with soya flour and mannitol.

4. The process as claimed in claim 2, wherein Streptomyces spec. DSM 3813 is cultured at 18 to 35°C. 35°C.

5. The process as claimed in one or more of claims 2 to 4, wherein Streptomyces spec. DSM 3813 is cultured at a pH of 5 to 8.5.

6. The process as claimed in one or more of claims 2 to 5, wherein a derivative is formed chemically from the compound prepared by fermentation.

7. The use of a compound of the formula I

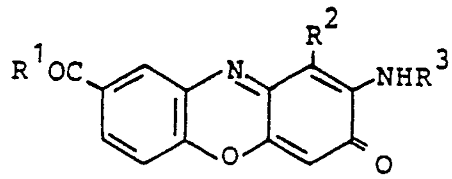

in which

$R^1$ is hydroxyl or ($C_1$—$C_5$)-alkoxy or amino,

$R^2$ is hydrogen or the group of the formula

$$—S—CH_2—\underset{\displaystyle NHR^4}{\underset{|}{CH}}—COR^5,$$

with $R^4$ as hydrogen or ($C_1$—$C_5$)-acyl and $R^5$ as hydroxyl or ($C_1$—$C_5$)-alkoxy, and

$R^3$ is hydrogen or ($C_1$—$C_5$)-acyl,

for the preparation of a medicine or diagnostic.

8. The use of a compound as claimed in claim 7 for the preparation of a medicine for treating fungal, protozoal and viral infections, and for the preparation of an anthelmintic, and as a lipoxygenase inhibitor.

9. A compound as claimed in claim 7, for use as an agent against fungi, viruses, protozoa and helminthes.

10. Streptomyces spec. DSM 3813 for a variant or mutant thereof, where this produces a compound of the formula I

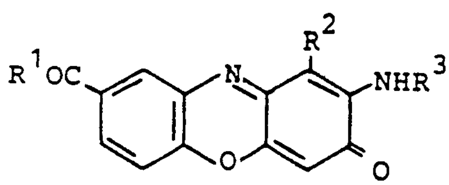

in which

$R^1$ is hydroxyl or ($C_1$—$C_5$)-alkoxy or amino,

$R^2$ is hydrogen or the group of the formula

$$—S—CH_2—\underset{\displaystyle NHR^4}{\underset{|}{CH}}—COR^5,$$

with $R^4$ as hydrogen or ($C_1$—$C_5$)-acyl and $R^5$ as hydroxyl or ($C_1$—$C_5$)-alkoxy, and

$R^3$ is hydrogen or ($C_1$—$C_5$)-acyl.

**Claims for the Contracting States: AT ES GR**

1. A process for the preparation of a compound of the formula I

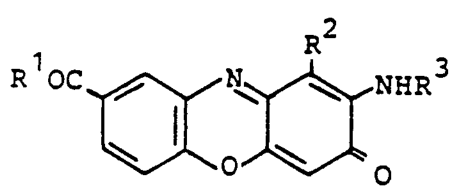

in which

$R^1$ is hydroxyl or ($C_1$—$C_5$)-alkoxy or amino,

$R^2$ is hydrogen or the group of the formula

$$—S—CH_2—\underset{\displaystyle NHR^4}{\underset{|}{CH}}—COR^5,$$

with $R^4$ as hydrogen or ($C_1$—$C_5$)-acyl and $R^5$ as hydroxyl or ($C_1$—$C_5$)-alkoxy, and

$R^3$ is hydrogen or ($C_1$—$C_5$)-acyl,

which comprises

a) culturing Streptomyces spec. DMS 3813 on a nutrient medium until a compound of the formula I in which

1) $R^1$ is hydroxyl, $R^2$ is

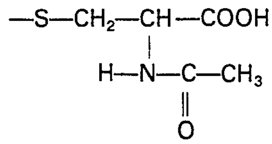

and $R^3$ is hydrogen, or

2) $R^1$ is hydroxyl, $R^2$ is hydrogen and $R^3$ is acetyl, or

3) $R^1$ is amino and $R^2$ and $R^3$ are hydrogen, has accumulated in the nutrient medium and if appropriate

b) isolating the compound mentioned and forming derivatives.

2. The process as claimed in claim 1, wherein Streptomyces spec. DS 3813 is cultured in a nutrient solution with soya flour and mannitol.

3. The process as claimed in claim 1 or 2, wherein Streptomyces spec. DSM 3813 is cultured at 18 to 35°C.

4. The process as claimed in one or more of claims 1 to 3, wherein Streptomyces spec. DSM 3813 is cultured at a pH of 5 to 8.5.

5. The process as claimed in one or more of claims 1 to 4, wherein a derivative is formed chemically from the compound prepared by fermentation.

6. The use of a compound obtainable as defined in claim 1 for the preparation of a medicine or diagnostic.

7. The use of a compound obtainable as defined in in claim 1 for the preparation of a medicine for treating fungal, protozoal and viral infections, and for the preparation of an anthelmintic, and as a lipoxygenase inhibitor.

8. A compound obtainable as defined in claim 1, for use as an agent against fungi, viruses and helminthes.